# DEMANDE DE BREVET EUROPEEN

(11) **EP 1 649 859 A1**
(43) Date de publication de la demande: **26.04.2006**
(21) Numéro de dépôt: 04292515.6
(22) Date de dépôt: 22.10.2004
(51) Int. Cl.: A61K 31/70, A61K 31/00, A61K 48/00, A61P 35/00, A61K 45/06, A61N 1/02

(54) **Produits pharmaceutiques contenant au moins un principe actif anticancereux peu diffusible et un principe actif immunostimulant sous forme d'oligodésoxynucléotide**

(71) Demandeur: Institut Gustave Roussy, 94800 Villejuif (FR); CENTRE NATIONAL DE LA RECHERCHE SCIENTIFIQUE, 75016 Paris Cédex (FR); UNIVERSITE RENE DESCARTES, (PARIS V), F-75006 Paris (FR); Oligovax, 75014 Paris (FR)
(72) Inventeur: Robert, Caroline, 92260 Fontenay-aux-Roses (FR); Mir, Lluis, 91370 Verrieres-le-Buisson (FR); Carpentier, Antoine, 75007 Paris (FR)
(74) Mandataire: Goulard, Sophie

(57) **Abrégé**

Lesdits produits contiennent au moins un principe actif anticancéreux peu diffusible et un principe actif immunostimulant sélectionné dans le groupe constitué par des ligands de TLR en tant que préparation combinée pour une utilisation simultanée, séparée ou étalée dans le temps, pour le traitement des tumeurs bénignes ou malignes et plus particulièrement des métastases, chez un sujet atteint d'une tumeur et soumis à une électroperméabilisation au niveau de ladite tumeur.

## Description

La présente invention est relative à des produits utilisés en combinaison, en vue d'un traitement efficace des tumeurs et plus particulièrement des métastases.

Les trois principaux traitements anticancéreux sont la chirurgie, la radiothérapie et la chimiothérapie. Cependant, l'efficacité de ces thérapies est souvent insuffisante dans le cas des cancers métastatiques en raison de la taille et du nombre des tumeurs. Il est donc nécessaire de disposer de nouvelles approches thérapeutiques.

Ainsi, l'efficacité des thérapeutiques conventionnelles chirurgicales ou à visée cytolytique (chimiothérapie et radiothérapie) reste très limitée dans de nombreux cancers. Pour les glioblastomes par exemple, dont le traitement repose principalement sur l'exérèse chirurgicale et l'irradiation cérébrale locale, la médiane de survie est de l'ordre de 12 mois. Une chimiothérapie complémentaire allonge la survie chez les patients de moins de 60 ans, mais de façon très modeste, de moins de 3 mois. Sous ce triple traitement, la médiane de survie reste inférieure à 15 mois (Behin et al, Lancet 2003).

L'immunothérapie dans le traitement des cancers vise à stimuler le système immunitaire afin de détruire les cellules tumorales. Différentes approches peuvent être utilisées : vaccination contre un ou plusieurs antigènes tumoraux, amplification d'une réponse antitumorale préexistante (comme par exemple amplification de lymphocytes cytotoxiques infiltrant la tumeur), utilisation d'anticorps monoclonaux dirigés contre un antigène tumoral ou de cellules dendritiques chargées avec des extraits ou des antigènes tumoraux (revue par Turtle CJ et al. Dendritic cells in tumor immunology and immunotherapy. Curr Drug Targets. 2004 5:17-39 ; Chaput N et al, Exosome-based immunotherapy. Cancer Immunol Immunother. 2004 ; 53:234-9 ; Parish CR.et al. Cancer immunotherapy: the past, the present and the future. Immunol Cell Biol. 2003 81:106-13).

Malheureusement, alors que les modèles animaux sont souvent convaincants, les essais cliniques d'immunothérapie chez l'homme n'ont pas encore trouvé leur place dans l'arsenal thérapeutique et les incertitudes concernant l'optimisation de ces paramètres sont encore très nombreuses. En particulier, le type d'antigène tumoral ainsi que sa voie d'administration sont encore des paramètres en cours d'évaluation.

Une autre stratégie consiste à activer le système immunitaire de façon large afin de stimuler l'immunité innée et d'amorcer ou d'amplifier une réponse immunitaire spécifique. Parmi ces agents immunostimulants, on peut citer, entre autres, les extraits bactériens (Jaeckle K. *A. et al.* (1990), *J. Clin. Oncol.* 8(8) pp 1408-18), l'IL-2 (Herrlinger U*. et al.* (1996), *J Neurooncol.* 27(3) pp 193-203), l'IL-12 (Kishima *H. et al.* (1998), *Br. J Cancer* 78(4) pp 446-53 ; Jean W. *C. et al.* (1998), *Neurosurgery* 42(4) pp 850-6), l'ADN bactérien (MY-1) (Tokunaga T. *et al.* (1984), *JNCI72* pp 955-62), ou le poly (I,C) (Ewel, C. H. et al (1992). Canc. Res. 52:3005).

Les propriétés immunostimulantes de l'ADN ont été découvertes en 1984, lorsqu'il a été observé que l'ADN de *Mycobacterium tuberculosis* activait in *vitro* et *in vivo* les cellules NK [Tokunaga T, et al. *J Natl Cancer Inst* 1984; 72: 955-962]. Plusieurs études ont ensuite montré que ces propriétés immunostimulantes étaient généralement en rapport avec des motifs 5'-CG non méthylés, motifs sous-représentés dans l'ADN des vertébrés [Krieg AM et al. *Nature* 1995; 374: 546-549 ; Yamamoto S et al *J Immunol* 1992; 148: 4072-4076 ; Demandes US 2004-0006034 ; 2003-0212029 ; Brevets US 6,653,292 ; U.S. 6,239,116 ; U.S. 6,207,646 ; U.S 6,194,388 ; US 6,429,199, US 6,406,705, US 6,218,371, US 6,214,806, US 6,218,371, 6,727,230, Demandes Internationales PCT WO 01/51500, WO 03/035695, Demande de Brevet Européen n° 1 162 982]. Les oligodésoxynucléotides synthétiques (ODN) contenant de tels motifs (CpG-ODN) gardent des propriétés immunostimulantes marquées, particulièrement les ODN phosphorothioates qui sont résistants aux nucléases.

La stimulation du système immunitaire par des CpG-ODN ou de l'ADN bactérien requiert leur pénétration intracellulaire, probablement par endocytose. Après dégradation dans le milieu acide des endosomes, les motifs CpG sont reconnus de façon spécifique par un membre de la famille des Toll-like receptors, le TLR9, exprimé au sein des endosomes. L'activation intracellulaire après liaison au TLR9 dépend des protéines MyD88, IRAK et TRAF6, qui à leur tour activent les voies des MAP kinase et du NFκB [Hacker H, *Curr Top Microbiol Immunol* 2000 ; 247: 77-92 ; Hemmi H, et al. *Nature* 2000; 408, 740-745 ; Takeshita Fet al, *J Immunol* 2001; 167, 3555-8]. Chez l'homme l'expression de TLR9 prédomine au niveau des lymphocytes B et des cellules dendritiques plasmocytoïdes (pDC), alors que l'expression est plus large chez la souris incluant la lignée myéloïde et la microglie[Hornung V, Rothenfusser Set al, *J Immunol* 2002; 168, 4531-4537].

Les lymphocytes B sont activés par les CpG-ODN, résultant dans la sécrétion de cytokines comme par exemple l'IL-6 ou l'IL-10, la prolifération cellulaire, l'inhibition de l'apoptose induite par divers agents et la sécrétion d'immunoglobulines [revue par Klinman DM, Nat Rev Immunol 2004 ; 4: 249-59 ; Krieg AM, Curr Oncol Rep 2004 ; 6: 88-95 ; Carpentier AF et al, *Front Biosci* 2003 ; 8: E115-27].

L'activation des pDC humaines entraîne leur maturation, la sécrétion de nombreuses cytokines comme le TNFα, l'interféron alpha ou gamma, l'IL-6 ou l'IL-12 et l'expression de molécules de co-stimulation (CD40, CD80, CD86) et de CCR7, un récepteur contrôlant la migration vers les zones T ganglionnaires. Alors que les pDC sont directement activées par les CpG-ODN, l'activation des cellules dendritiques myéloïdes humaines semble indirecte par le biais de cytokines [revue par Klinman DM, Nat Rev Immunol 2004 ; 4: 249-59 ; Krieg AM, Curr Oncol Rep 2004 ; 6: 88-95]. L'activation des monocytes par les cytokines sécrétées peut induire, entre autres, la sécrétion de CXCL 10 qui favorise la réponse immunitaire antitumorale et qui possède également des propriétés anti-angiogéniques (Strieter RM et al (1995), J Biol Chem, 270 ; 27348-57).

L'activation des lymphocytes T et des cellules NK par les CpG-ODN dépend des cytokines sécrétées par les DCs (cellules dendritiques) et les lymphocytes B. La sécrétion d'IL-12 et d'IFN gamma oriente la réponse vers le profil Th1, et peut même transformer une réponse Th2 en Th1. De plus, les DCs peuvent alors activer les lymphocytes CD8, indépendamment des lymphocytes CD4 helper [revue par Klinman DM, Nat Rev Immunol 2004 ; 4: 249-59 ; Krieg AM, Curr Oncol Rep 2004; 6: 88-95].

L'activité biologique d'un oligonucléotide dépend de nombreuses variables, encore imparfaitement connues, comme par exemple les séquences des nucléotides entourant le motif CpG ou le nombre de motifs CpG. Quelques oligonucléotides immunostimulants sans motifs CpG ont même été décrits (par exemple dans la Demande US 2004-0006032). La modification chimique du squelette naturellement constitué dans l'ADN par les liaisons phophodiesters (sensibles aux nucléases), joue un rôle primordial à la fois en améliorant 1a stabilité de l'ODN mais aussi en modifiant ses propriétés immunostimulantes. Plusieurs types d'oligonucléotides stabilisés ont ainsi été créés (Iyer RP (1999) Curr Opinion Mol Therap 1 ; 344-358). Les CpG-ODN les plus fréquemment utilisés sont les oligodésoxynucléotides phosphorothioates, ou des oligodésoxynucléotides mixtes phosphoro-thioates/phosphodiesters (par exemple extrémités phosphorothioates/centre phospho-diester ; ou des ODN phosphorothioates sauf les liaisons cytosine-guanosine qui sont phosphodiesters). Les oligodésoxynucléotides synthétisés peuvent également être purifiés en fonction de leur stéréoisomérie pour modifier ou améliorer leur activité biologique.

L'activité immunostimulante d'oligonucléotides synthétiques très modifiés, en raison de la présence de bases purines ou pyrimidines non naturelles (Kandimalla ER et al, Nucleic acids Research, 2003, 31 ; 2393-2400 ; Demandes US 2003-0181406 ; 2002-0137714 ; Brevet US 6,562,798 ; Demande US 2003-0186912), de molécules chimériques ADN/ARN ou d'ODN-linker-ODN (Demande US 2004-0052763 ; Bhagat L et al, BBRC, 2003, 300 ; 853-861 ; Demandes US 2003-0225016 ; 2003-0199466 ; 2003-0175731) ou l'adjonction de ligands non oligonucléotidiques a également été rapportée (revue par Uhlman et Vollmer (2003), 6, 204-217).

Les activités biologiques précises de chacun de ces oligonucléotides ne sont pas encore parfaitement connues. Trois familles de CpG-ODN ont déjà été caractérisées [revue par Klinman DM, Nat Rev Immunol 2004; 4: 249-59 ; Krieg AM, Curr Oncol Rep 2004; 6: 88-95]. La plus classique, dénommée "type B " (ou " K "), se caractérise par une forte activation des lymphocytes B et des cellules dendritiques, mais une faible sécrétion d'interféron alpha par les pDC. Les ODN de " type A " (ou "D") se caractérisent par une faible activation des lymphocytes B mais une forte activation des NK et une sécrétion d'interféron alpha par les pDC. Les ODN de type C, les plus récemment décrits, combinent les propriétés des 2 types précédents [revue par Klinman DM, Nat Rev Immunol 2004; 4: 249-59 ; Krieg AM, Curr Oncol Rep 2004; 6: 88-95].

Le fort pouvoir immunostimulant des CpG-ODN (et par extension des ligands de TLR et plus particulièrement les ligands de TLR9) ouvre plusieurs stratégies thérapeutiques dans les cancers. Leur utilisation permet en effet d'activer les deux composantes de la réponse immune :
- L'amorçage de la réponse immune : par la stimulation directe des cellules dendritiques et la sélection par le système immunitaire d'un antigène adéquat avec une réponse immune de type Th1.
- L'efficacité de la réponse immune : l'activation des macrophages et des cellules NK permet d'accroître la cytotoxicité anti-tumorale, soit directe, soit par le biais des anticorps (antibody-dependant cell cytotoxicity ou ADCC). De plus, grâce à la sécrétion locale d'IFN gamma, l'expression généralement faible du CMH I par les cellules tumorales peut être induite, les rendant plus sensibles à la cytotoxicité des lymphocytes T.

Ces activités biologiques multiples ont ainsi permis le développement de plusieurs stratégies dans les cancers :
1. Les CpG-ODN utilisés en monothérapie par voie locale (intra ou péri-tumorale) ou générale (= systémique) peuvent induire le rejet de tumeurs dans presque tous les modèles de tumeurs [Carpentier AF, Auf G, Delattre JY. CpG-oligonucleotides for cancer immunotherapy: review of the literature and potential applications in malignant glioma. *Front Biosci* 2003; 8: E115-27]. Par exemple, dans un modèle de gliome intracérébral (CNS1), plus de 85 % des rats furent guéris par une seule injection locale de CpG-ODN [Carpentier AF, Xie J, Mokhtari K , Delattre JY, *Clin Cancer Res* 2000; 6, 2469-2473]. La plupart des études ont souligné le rôle conjoint du système immunitaire inné non spécifique et du système immunitaire spécifique dans le rejet tumoral. La déplétion *in vivo* des cellules NK diminue ou abolit les effets anti-tumoraux des CpG-ODN [Carpentier AF, Chen L, Maltonti F, Delattre JY. *Cancer Res* 1999; 59, 5429-5432]. Les lymphocytes CD8+ sont nécessaires dans la plupart des modèles animaux, alors que la déplétion en cellules CD4 semble avoir moins d'impact [Lanuti M, Rudginsky S, Force SD, et al. *Cancer Res* 2000; 60, 2955-2963 ; Kawarada Y, Ganss R, Garbi N, et al. *J Immunol* 2001; 167, 5247-5253]. Quoi qu'il en soit, l'existence d'une mémoire immunitaire à long terme, rapportée par l'ensemble des modèles suggère un rôle pour les lymphocytes CD4 mémoire. De façon intéressante, il a été rapporté que les pDC infiltrant des tumeurs ORL chez l'homme étaient moins sensibles aux CpG-ODN de type A que les pDC sanguines du même patient. Cette résistance s'expliquerait en partie par la sécrétion locale d'IL-10 par les cellules tumorale [Hartmann E, Wollenberg B, Rothenfusser S, et al. Cancer Res. 2003; 63: 6478-87]. Dans un modèle animal, la combinaison de CpG et d'anticorps anti-IL-10 s'est d'ailleurs révélée synergique [Vicari AP, Chiodoni C, Vaure C, et al. J Exp Med 2002; 196, 541-9].
2. Les CpG-ODN peuvent également être utilisés pour la fabrication de vaccins, étant des adjuvants très efficaces avec la plupart des antigènes, sauf si ceux-ci sont de nature purement polysaccharidique [Krieg AM. Annu Rev Immunol 2002; 20:709-60 ; Krieg AM, Curr Oncol Rep 2004; 6: 88-95]. Les CpG-ODN se sont révélés être les adjuvants les plus efficaces pour l'induction des réponses immunitaires Th1 cytotoxiques avec lymphocytes T CD4 et CD8 spécifiques de l'antigène [Zwaveling S, Ferreira Mota SC, Nouta J, et al. J Immunol 2002; 169 : 350-8 ; Miconnet I, Koenig S, Speiser D, et al. *J Immunol.* 2002; 68, 1212-1218]. La conjugaison directe entre l'antigène et les CpG-ODN permet de réduire la quantité d'antigène nécessaire [Tighe H, Takabayashi K, Schwartz D, et al. J Allergy Clin Immunol. 2000; 106: 124-34.]. L'efficacité des CpG-ODN est encore améliorée par la combinaison avec d'autres adjuvants comme par exemple l'hydroxyde d'aluminium, le QS21, le MPL ou le GMCSF [Miconnet I, Koenig S, Speiser D, et al. CpG are efficient adjuvants for specific CTL induction against tumor antigen-derived peptide. J *Immunol.* 2002; 68, 1212-1218 ; Kim SK, Ragupathi G, Cappello S, et al. Effect of immunological adjuvant combinations on the antibody and T-cell response to vaccination with MUC1-KLH and GD3-KLH conjugates. Vaccine. 2000; 19: 530-7 ; Liu HM, Newbrough SE, Bhatia SK, et al. Immunostimulatory CpG oligodeoxynucleotides enhance the immune response to vaccine strategies involving granulocyte-macrophage colony-stimulating factor. *Blood* 1998; 92, 3730-3736]. Malheureusement, les cibles antigéniques sont rarement déterminées dans les cancers, limitant l'intérêt pratique de telles stratégies thérapeutiques. De plus la sélection spontanée d'un clone n'exprimant pas l'antigène permettrait à la tumeur d'échapper à un tel traitement.
3. La combinaison de CpG-ODN et d'anticorps monoclonaux est également intéressante. Elle augmente la cytotoxicité médiée par les anticorps (ADCC) grâce à la stimulation des macrophages et des cellules NK ; plusieurs essais cliniques utilisant cette association ont débuté récemment, avec de l'Herceptin® dans les cancers du sein ou du Rituxan® dans les lymphomes non-hodgkiniens. Cette combinaison peut aussi utiliser les CpG-ODN comme inducteurs au niveau de la cellule tumorale de l'expression d'une molécule contre laquelle un anticorps monoclonal existe. Les CpG-ODN induisent l'expression du CD25 (IL2R) à la surface d'une lignée lymphomateuse B, rendant ainsi ces cellules plus sensibles à l'action d'une immunotoxine anti-CD25 [Decker T, Hipp S, Kreitman RJ, et al. *Blood* 2002; 99, 1320-1326].
4. L'utilisation des CpG-ODN pour *stimuler in vitro ou in vivo* les cellules dendritiques a été décrite. Cette stimulation peut se faire en association avec d'autres adjuvants immunitaires ou cytokines, avec divers antigènes, ou extraits tumoraux (Krieg AM. Annu Rev Immunol 2002; 20:709-60 ; Krieg AM, Curr Oncol Rep 2004; 6: 88-95).
5. Enfin l'utilisation des ligands de TLR9 ou des CpG-ODN pour l'immunothérapie des cancers peut être combinée avec d'autres thérapies, notamment la chirurgie, la radiothérapie, les chimiothérapies, d'autres immunothérapies et des thérapies différenciantes.

A notamment été décrite une utilisation combinée de la chimiothérapie par voie systémique et des CpG-ODN (par voie locale ou systémique). Cette combinaison permet, en théorie, de diminuer la masse tumorale à éliminer par le système immunitaire et d'induire le relargage d'un grand nombre d'antigènes tumoraux qui serviront à amorcer le système immunitaire. Des résultats positifs de telles approches ont été rapportés par plusieurs auteurs dans quelques modèles tumoraux et avec quelques chimiothérapies comme le cyclophosphamide et le topotecan (Carpentier 2003 précité ; Weigel BJ, et al., Clin. Cancer Res., 2003, 9, 8, 3105-14 ; Balsari A. et al., Eur. J. Cancer, 2004, 40, 8, 1275-81).

Cette combinaison n'est cependant pas efficace dans tous les modèles tumoraux. En particulier, certaines tumeurs sont peu ou pas sensibles aux chimiothérapies, limitant ainsi l'utilité clinique d'une combinaison CpG-ODN et chimiothérapie conventionnelle.

L'un des obstacles à surmonter pour obtenir une bonne efficacité des CpG-ODN dans les cancers, est l'induction d'une immunité spécifique. En effet l'activation par les CpG-ODN de l'immunité non-spécifique est locale et ne persiste que pendant la durée du traitement. La capacité des CpG-ODN à induire une bonne réponse immunitaire spécifique capable d'agir à distance et dans la durée est donc déterminante pour les cancers métastatiques. Ceci est illustré par les résultats préliminaires d'un essai clinique dans les mélanomes ou des réponses locales furent observées, mais sans réponse à distance (pas d'effet systémique) (Trefzer U. et al., 2^{nd} International Symposium « Activating immunity with CpG oligos », Amelia Island, Florida, October 7-10, 2001).

De manière surprenante, les Inventeurs ont trouvé qu'une destruction tumorale par une technique d'électrochimiothérapie associée à une stimulation adaptée du système immunitaire, conduit à une thérapie antitumorale avec effet à distance (effet systémique) particulièrement efficace, notamment en ce qui concerne les métastases.

Les Inventeurs ont en particulier trouvé que la combinaison de ligands (naturels ou synthétiques) de TLR (Toll-like Receptor), en particulier des oligodésoxynucléotides (ODN) immunostimulants et d'un protocole de chimiothérapie optimisé permet d'obtenir une action efficace sur les métastases.

La présente invention a, en conséquence, pour objet des produits contenant au moins un principe actif anticancéreux peu diffusible et un principe actif immunostimulant sélectionné dans le groupe constitué par des ligands de TLR, en tant que préparation combinée pour une utilisation simultanée, séparée ou étalée dans le temps, pour le traitement des tumeurs bénignes ou malignes, notamment les tumeurs solides, et plus particulièrement des métastases, chez un sujet atteint d'une tumeur et soumis à une électroperméabilisation au niveau de ladite tumeur.

En variante, l'électroperméabilisation est réalisée au niveau d'un tissu sain sélectionné de manière appropriée.

Dans un tel cas, la préparation combinée desdits produits comprend en outre des antigènes par exemple sous forme d'antigènes purifiés spécifiques d'une tumeur, d'extraits tumoraux, de cellules tumorales irradiées ou modifiées, lesdits antigènes étant de préférence administrés localement.

Selon cette variante de l'invention, l'électroperméabilisation effectuée sur un tissu sain, a l'avantage de promouvoir localement le recrutement de cellules immunitaires.

L'électrochimiothérapie (ECT) (Mir et al, 1991a, Belehradek et al, 1991) combine *l'administration in situ* de molécules anticancéreuses non perméantes (ou non diffusible) ou peu perméantes (ou peu diffusible) et l'application d'impulsions électriques perméabilisantes. Parmi les molécules anticancéreuses, qui ont été mises en oeuvre dans le cadre d'une ECT, on peut citer la bléomycine, une molécule non perméante, qui ne diffuse pas à travers la membrane de la cellule, déjà utilisée en chimiothérapie classique (Orlowski et al, 1988; Poddevin et al, 1991; Gehl et al, 1999) et le cisplatine et les autres sels du platine ayant des propriétés cytotoxiques (Sersa et al, 1995; Gehl et al, 1999; Sersa et al, 2003). D'autres molécules peuvent également être mises en oeuvre dans le cadre d'une ECT, telles que des substances pro-apoptotiques, anti-angiogéniques, différenciantes ou immunostimulantes ; ainsi l'ECT a été utilisée pour améliorer la transfection d'oligonucléotides antisens ou de plasmides (Ivanov MA et al., J. Gene Mede, 2003, 5, 893-899, Brevets US 5,547,467, 5,749,847 et 6,763,264) ou l'administration de molécules immunostimulantes comme l'interleukine 12 (Kishida T et al., Mol. Ther., 2003, 8, 738-745),

L'électroperméabilisation des cellules exposées à des impulsions électriques est un phénomène connu depuis une trentaine d'années. Il résulte de la différence de potentiel transmembranaire générée par un champ électrique externe à la surface des cellules exposées à ce champ. Lorsque cette différence de potentiel transmembranaire dépasse une certaine valeur seuil (Teissié et al, 1993), pendant un temps suffisamment long (Kotnik et al, 2003), la perméabilisation transitoire et réversible des cellules peut être obtenue, sans entraîner de lésions tissulaires. L'intensité du champ électrique appliqué dépend du voltage et de la distance entre les électrodes. Il peut varier de 20 à 2000 V/cm. Les impulsions électriques peuvent varier de 3 à 8 ; elles ont généralement une durée de 100 µs et sont délivrées à une fréquence de 1 à 5000 Hz.

L'électroperméabilisation des cellules d'une tumeur peut être obtenue, par exemple, par l'exposition de cette tumeur à 3-8 impulsions électriques de 100 microsecondes et 1300 V/cm délivrées à la fréquence de répétition de 1 Hz par l'intermédiaire de deux électrodes posées sur la peau de part et d'autre du nodule tumoral à traiter. De toute évidence, il s'agit d'un traitement local, restreint au volume compris entre les électrodes et soumis à une intensité de champ électrique supérieure à la valeur seuil qui permet de perméabiliser les cellules comprises dans ce volume : la bléomycine, administrée préalablement, va pénétrer uniquement dans les cellules électroperméabilisées et seules ces dernières seront effectivement tuées.

Selon un mode de réalisation avantageux desdits produits, les ligands de TLR sont des oligodésoxynucléotides (ODN) immunostimulants, que leur structure soit naturelle, synthétique ou chimérique.

Selon une disposition avantageuse de ce mode de réalisation, les ligands de TLR sont plus précisément des ligands de TLR9 et plus particulièrement des CpG-ODN immunostimulants.

Conformément à l'invention, lesdits CpG-ODN immunostimulants sont sélectionnés dans le groupe constitué par un oligodésoxynucléotide stabilisé qui comprend au moins un motif quadramérique de formule X₁-CG-X₂, dans laquelle X₁ et X₂ sont identiques ou différents et représentent T ou A.

Selon une disposition avantageuse de ce mode de réalisation, ledit CpG-ODN est de préférence sélectionné dans le groupe constitué par un oligodésoxynucléotide qui comprend au moins une séquence quadramérique sélectionnée dans le groupe constitué par : TCGA, ACGT, ACGA et TCGT.

Selon une modalité avantageuse de cette disposition, ledit CpG-ODN est de préférence sélectionné dans le groupe constitué par un oligodésoxynucléotide qui comprend au moins une séquence hexamérique sélectionnée dans le groupe constitué par: **AACGTT, GACGTC, GACGTT, GTCGTT, TTCGAA, TACGTA, ATCGAT, TTCGTT** et **ATCGTT.**

Selon une autre disposition avantageuse de ce mode de réalisation, ledit CpG-ODN est de préférence sélectionné dans le groupe constitué par un oligonucléotide qui comprend au moins la séquence octamérique suivante : AACGTT-X₃X₄, dans laquelle X₃X₄ est AT, AA, CT ou TT et de préférence la séquence octamérique AACGTTAT.

De tels CpG-ODN stabilisés ainsi que leur procédé de préparation sont notamment décrits dans les Demandes française n° 99 03432 et Internationale PCT n° WO 00/56342.

Selon encore un autre mode de réalisation avantageux desdits produits, ledit CpG-ODN est le CpG-ODN de séquence SEQ ID NO:1.

Conformément à l'invention, les oligonucléotides immunostimulants peuvent être utilisés sous forme de simple-brin ou de double-brin, peuvent comprendre plusieurs séquences immunostimulantes adjacentes ou non. Ils peuvent comprendre également d'autres séquences biologiquement actives, comme des séquences anti-sens ou des aptamères. Les oligonucléotides stabilisés peuvent être couplés par des liaisons covalentes, ioniques ou faibles, à une ou plusieurs autres molécules susceptibles d'augmenter leur affinité tumorale, de modifier leur activité biologique ou d'augmenter leur activité immunostimulante.

Au sens de la présente invention, le principe actif anticancéreux est une substance habituellement utilisée en chimiothérapie ; la chimiothérapie anticancéreuse repose sur l'utilisation de substances cytotoxiques appartenant à deux catégories de substances : des antimétabolites qui interfèrent notamment avec la synthèse des acides nucléiques (fluoro-uracile, méthotrexate, doxorubicine), des protéines (inhibiteurs de la topo-isomérase) ou bien d'autres processus métaboliques essentiels comme la mitose (vincristine) et des substances génotoxiques qui modifient la structure de l'ADN comme des agents scindants (bléomycine) ou intercalants, notamment des agents alkylants (cyclophosphamide, lomustine, témozolomide, dérivés du platine (cisplatine ou cis-diaminedichloroplatine, cDDP ou DDP], dérivés de nitrosourée (fotémustine ou [[(chloro-2-éthyl)-nitroso-3 uréido]-1 éthyl] phospho-nate de diéthyle-(RS)). L'efficacité de cette thérapie peut varier de façon importante d'un type de tumeur à l'autre et d'un individu à l'autre, en raison de différences dans la sensibilité des cellules tumorales à ces agents.

D'autres substances sont également utilisées en chimiothérapie anticancéreuse ; il s'agit de substances capables d'agir sur d'autres facteurs que ceux précisés ci-dessus et qui interviennent dans les cancers : substances cytolytiques (ou pro-apoptotiques), substances anti-angiogéniques et substances différenciantes.

Selon un mode de réalisation avantageux desdits produits, le principe actif anticancéreux est sélectionné dans le groupe constitué par les substances cytotoxiques (les antimétabolites et les substances génotoxiques).

Selon une disposition avantageuse de ce mode de réalisation, le principe actif anticancéreux est de préférence une substance génotoxique et de manière encore plus préférée de la bléomycine.

Selon encore un autre mode de réalisation avantageux desdits produits, les cellules de la tumeur sont de préférence électroperméabilisées ultérieurement à l'administration desdits produits, par exposition desdites cellules tumorales à des impulsions électriques de force ou d'intensité et de durée suffisantes pour permettre l'électroporation desdites cellules cancéreuses ; lesdites cellules peuvent également être électroperméabilisées préalablement à l'administration desdits produits.

Selon un autre mode de réalisation avantageux desdits produits, l'intensité du champ électrique appliqué varie entre 20 et 2 000 V/cm.

Selon une disposition avantageuse de ce mode de réalisation, l'intensité du champ électrique appliqué varie de préférence entre 500 et 1 500 V/cm et de préférence entre 800 et 1 300 V/cm.

Selon encore un autre mode de réalisation avantageux desdits produits, la fréquence des impulsions varie de 0,01 à 10 000 Hz.

Selon une disposition avantageuse de ce mode de réalisation, la fréquence des impulsions varie de préférence, de 1 à 5 000 Hz.

Selon encore un autre mode de réalisation avantageux desdits produits, la durée des impulsions varie de 10 µs à 10 s.

Selon une disposition avantageuse de ce mode de réalisation, la durée des impulsions varie de préférence de 50 µs à 500 ms, et de préférence de 100 µs à 1 ms.

Selon un autre mode de réalisation avantageux desdits produits, le principe actif anticancéreux est administré par voie intratumorale, locale ou systémique.

Selon un autre mode de réalisation avantageux desdits produits, le principe actif immunostimulant est administré par voie intratumorale, locale ou systémique.

Conformément à l'invention, lesdits produits sont administrés en une ou plusieurs fois ou en libération continue, notamment au moyen de micropompes osmotiques, ou associés à tout moyen physique ou chimique, notamment à des agents encapsulants comme les systèmes de dispersion colloïdaux et les polymères.

Également conformément à l'invention, lesdits produits sont en outre associés à un autre traitement, tel que la radiothérapie, une autre substance cytotoxique, une substance cytolytique, une substance anti-angiogénique, une substance différenciante, une substance immunostimulante autre qu'un ODN ou des produits issus de la thérapie cellulaire, comme des cellules dendritiques.

De manière avantageuse, les produits selon l'invention peuvent être utilisés dans le traitement des cancers, quels que soient leur nature et leur degré d'anaplasie et ils permettent d'obtenir, de manière surprenante, un effet curatif systémique, notamment sur les métastases

Ainsi, l'injection d'une simple dose hebdomadaire de CpG dans le volume tumoral traité par électrochimiothérapie (la première injection étant effectuée le lendemain de l'électrochimiothérapie) a permis non seulement d'améliorer les résultats locaux de l'électrochimiothérapie mais encore, de manière surprenante, d'obtenir des effets à distance. La démonstration de ces effets systémiques, obtenus par la combinaison de deux traitements locaux (l'électrochimiothérapie et l'injection locale de CpG), a été faite chez la souris et le rat inoculés avec deux tumeurs transplantées dans les deux flancs des animaux à trois jours d'intervalle. La plus grande des tumeurs (la première à avoir été transplantée) est ensuite traitée par électrochimiothérapie et reçoit l'injection de CpG, et l'évolution des deux tumeurs est ensuite poursuivie dans les jours qui suivent. Comme le montrent les tableaux et graphiques rapportés, avec la combinaison de l'électrochimiothérapie et des CpG, des effets anti-tumoraux importants (régression complète) ont été obtenus au niveau de la tumeur traitée et de la tumeur contralatérale.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre l'effet du traitement par électrochimiothérapie (J0) et des CpG-ODN (J1, 8, 15, 22) intratumoraux sur deux tumeurs LPB opposées (Moyenne±EC). A : évolution de la tumeur gauche (traitée). B : évolution de la tumeur droite (non traitée).
- la figure 2 est une représentation détaillée souris par souris de l'effet du traitement par électrochimiothérapie (J0) et CpG-ODN (J1, 8, 15, 22) intratumoral sur deux tumeurs LPB opposées. A : évolution de la tumeur gauche (traitée). B : évolution de la tumeur droite (non traitée). (S : souris).
- la figure 3 illustre l'effet de la combinaison ECT+CpG-ODN dans le modèle de tumeur B 160VA (7 souris par groupe).
- la figure 4 illustre l'effet de l'électroporation des CpG-ODN à J1, 8, 15, 22 en intratumoral sur deux tumeurs LPB opposées (Moyenne±EC). A : évolution de la tumeur gauche (traitée). B : évolution de la tumeur droite (non traitée).
- la figure 5 est une représentation par souris de l'effet de l'électroporation des CpG-ODN à J1, 8, 15, 22 en intratumoral sur deux tumeurs LPB opposées. A : évolution de la tumeur gauche (traitée). B : évolution de la tumeur droite (non traitée). (S : souris).
- la figure 6 représente le monitoring de la réponse TCD8 dans le ganglion inguinal homolatéral des souris immunisées, traitées et non traitées. A : visualisation des lymphocytes. B : isolement des TCD8. C : isolement des TCDS spécifiques de SIINFEKL.

Il doit être bien entendu, toutefois, que ces exemples sont donnés uniquement à titre d'illustration de l'objet de l'invention, dont ils ne constituent en aucune manière une limitation.

### EXEMPLE 1 : MATERIELS ET METHODES.

**Animaux :** des souris femelles C57BL/6 H-2^{b} de 8-20 semaines ont été utilisées pour l'établissement des modèles tumoraux. Ces souris provenant du Centre d'Elevage Janvier (Le Genest, St Isle, France) ont toutes été élevées dans le service du petit animal de l'Institut Gustave Roussy conformément au guide du Comité d'Ethique d'Expérimentation Animal. Les souris ont toutes été sacrifiées humainement par inhalation de CO₂.

Lignées cellulaires tumorales : les lignées LPB et B160VA ont été *cultivées in vitro* à 37°C sous 5% de CO₂. Les cellules LPB sont dérivées d'un sarcome induit par le méthylcholanthrène chez la souris C57BL/6. Elles ont été maintenues dans du milieu essentiel minimum (Gibco, Cergy-Pontoise, France) supplémenté avec 8% de sérum de veau foetal (Gibco) inactivé par la chaleur (une demi heure à 56°C) et 100U/ml de pénicilline et 100 µg/ml de streptomycine (Gibco). La lignée stable B160VA fournie généreusement par le Dr K.L. Rock (UMass Medical School, Worcester, MA, USA), dérive d'un mélanome murin transfecté avec le gène codant pour la protéine de poulet ovalbumine (OVA). Ces cellules ont été maintenues *in vitro* dans du milieu RPMI1640 (Gibco) supplémenté avec 8% de sérum de veau foetal (SVF), 5% de L-glutamine (Gibco), 5% de pyruvate de sodium (Gibco), 5% d'acides aminés non essentiels (Gibco) et 5% de pénicilline et streptomycine.

Modèles tumoraux : après la trypsination des cellules et l'inactivation de la trypsine (Gibco) avec du milieu complet, les cellules ont été récoltées dans du milieu essentiel minimum (MEM) pour les LPB et dans du RPMI1640 pour les B 1 60VA. Les cellules LPB et B 160VA (10⁶) ont été injectées en sous-cutanée dans le flanc gauche des souris. Trois jours plus tard à nouveau 10⁶ cellules ont été injectées dans le flanc droit des mêmes animaux. Puis les souris ont été réparties pour chaque expérience avant d'être traitées 8 jours ou 9 jours après la première injection, respectivement pour les tumeurs LPB et B160VA. La taille des tumeurs a été mesurée 3 fois par semaine à l'aide un pied à coulisse. Le volume tumoral a été déterminé selon la formule d'une ellipse *V=a*^{*2*}*bπl6* dans laquelle a représente la largeur et b la longueur perpendiculaire

**Anesthésie :** les souris ont été anesthésiées avec 150 µl d'un mélange de xylazine (Bayer Pharma, Puteaux, France) à 12,5 mg/Kg et de kétamine (Parke Davis, Courbevoie, France) à 125 mg/Kg injecté en intrapéritonéal avant d'être traitées par électroporation ou électrochimiothérapie.

**Electrochimiothérapie :** 10 µg de bléomycine (Roger Bellon, Neuilly, France) dans 100 µl de NaCl 0,9% ont été injectés en iv dans le sinus rétro orbitaire des souris. Quatre minutes après l'injection, les impulsions électriques ont été délivrées sur la masse tumorale à l'aide d'un générateur relié à une console informatique (prototype). L'électroporation a été réalisée au moyen de deux électrodes espacées de 5 mm placées de part et d'autre de la tumeur, et délivrant 8 impulsions électriques de 100 µs à 1300 V/cm et 5000 Hz de fréquence. L'application systématique d'un gel sur la tumeur avant l'électroporation et le contrôle graphique de chaque électroporation permettaient de s'assurer de la bonne distribution du courant dans la tumeur.

**Immunothérapie :** la séquence simple brin de l'oligodéoxinucléotide contenant plusieurs motifs CpG non méthylés (5'-TAAACGTTATAACGTTATGACGTCAT-3') (SEQ ID NO:1). Cinquante micro-grammes d'oligodéoxinucléotides CpG dilués dans 100 µl de NaCl 0,9% ont été injectés en intratumoral le lendemain de l'ECT, une fois par semaine pendant 4 semaines.

**Essai de cytotoxicité *in vitro :*** une lignée fibroblastique de poumon de hamster chinois, DC3F, a été maintenue in vitro comme décrit précédemment pour la lignée LPB. Les cellules ont été récoltées afin de les re-suspendre à une concentration de 2,2.10⁷/ml dans du MEM-S (MEM sans calcium, Gibco). Des aliquotes de 10⁶ cellules/45 µl ont été mélangés à 5 µl de NaCl 0,9% ou 5 µl de CpG variant d'une concentration de 5 à 60 µM. Le mélange de 50 µl a ensuite été placé entre 2 électrodes espacées de 2 mm et aussitôt le choc électrique a été délivré (8 impulsions de 100 µs pour un champ électrique de 1000 V/cm et une fréquence de 1 Hz). Après 5 minutes de repos, les cellules ont été diluées dans du MEM complet et mises en culture en triple dans des boîtes de pétri de 6cm de diamètre (500 cellules/ml). La prolifération cellulaire a été estimée après 5 jours de culture. Pour cela les cellules ont d'abord été fixées avec du formaldéhyde pendant 20 minutes puis colorées avec du cristal violet pendant 15 minutes.

Analyse de la réponse immunitaire : Un modèle de tumeur a été créé en injectant 5.10⁵ cellules B16OVA pour 50 µl de RPMI1640 dans la cuisse droite des souris. Au bout de 14 jours, la tumeur mesurait 5 mm environ et était traitée par ECT (J0) suivi de l'immunothérapie par CpG à J1. Par ailleurs 3 souris ont été immunisées par injection de peptide directement dans le coussinet plantaire à J1: 45 µg de peptide SIINFEKL (Eurogentec) dérivé de l'ovalbumine mixé dans 50 µg d'ODN-CpG. Sept jours après ce traitement toutes les souris ont été sacrifiées afin de prélever les ganglions inguinaux. Les ganglions ont été écrasés mécaniquement dans des filtres de 100 µm et les cellules ganglionnaires ont été récoltées dans du RPMI1640. Dix mille cellules/puits ont été incubées dans une plaque de 96 puits avec du RPMI1640 supplémenté avec 1% de sérum murin, en présence ou en absence de peptide OVA, afin de réaliser une stimulation *in vitro.* Après 72 heures de culture à 37°C sous 5% de CO₂ le surnageant a été prélevé afin d'évaluer la sécrétion d'interféron γ par un test ELISA (kit OptEIA™ de BD Pharmingen). Une partie des cellules (1-2.10⁶) a été marquées avec le tétramère H-2^{b}/SIINFEKL couplé au PE (Beckman Coulter, Fullerton, CA) dilué au 1/100^{ème} pendant une demi heure à température ambiante et à l'obscurité, puis avec un anti-CD3-FITC et un anti-CD8-APC (BD Pharmingen, San Diego, CA) dilués au 1/100^{ème} pendant le même temps d'incubation. Les cellules ont ensuite été lavées une fois dans du PBS 1X et fixées dans 200 µl de paraformaldéhyde 1% avant d'être analysées au cytofluorimètre FACScalibur avec le logiciel CellquestPro.

Statistiques : une analyse de variance avec une comparaison multiple selon le test de Kruskal-Wallis a été réalisée pour les expériences *in vivo* et *in vitro.* Les moyennes avec les écart-types unilatéraux diffèrent significativement pour une valeur z supérieur à 1,96. Chaque test avait un p global inférieur à 0,05 permettant de comparer les groupes entre eux.

### EXEMPLE 2 : RESULTATS

### Activité antitumorale de la combinaison ECT et CpG-ODN (CpG) :

### • Modèle de fibrosarcome LPB

### - Analyse par groupe :

Pour tester l'hypothèse d'un effet thérapeutique systémique de la combinaison ECT-CpG, nous nous sommes placés dans un modèle de double tumeur. 10⁶ cellules LPB ont été implantées en sous-cutané dans le flanc gauche d'abord, puis 3 jours après à nouveau 10⁶ cellules dans le flanc droit des mêmes souris. Seule la tumeur de gauche a été traitée à J0 par EP ou ECT et à J1, J8, J15 et J22 par NaCl 0,9% ou CpG en intratumoral comme décrit dans la section matériels et méthodes. L'effet du traitement a été observé sur les 2 tumeurs.

En premier lieu des effets locaux du traitement (tumeurs gauches) sont traités, puis dans un second lieu des effets à distance (tumeurs droites) :

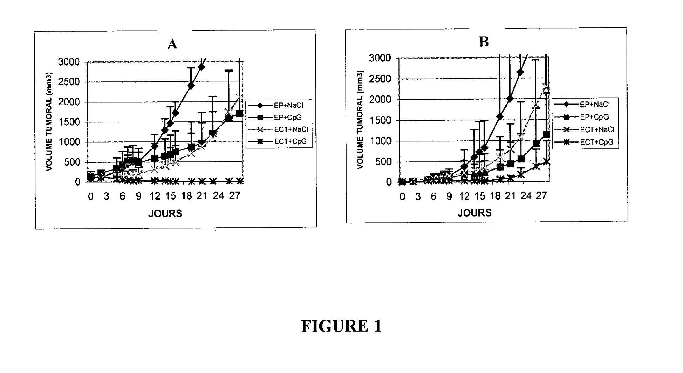

Les animaux ont été suivis pendant 30 jours après le traitement. Le groupe ayant reçu le traitement combiné ECT+CpG présentait 100% de régression complète des tumeurs traitées à J28 (Fig. 1A). En revanche lorsqu'un seul traitement était appliqué à savoir ECT seule ou CpG seuls, la croissance des tumeurs traitées a seulement été ralentie par rapport au groupe contrôle EP+NaCl (Fig. 1A). L'évolution des tumeurs est statistiquement différente entre le groupe ECT+CpG et les autres groupes (z=4,18 ; z=2,62 ; z=2,46 avec les groupes respectifs EP+NaCl, EP+CpG et ECT +NaCl à J28).

L'effet antitumoral à distance a été évalué par 1a mesure des tumeurs controlatérales (Fig. 1B). Une stabilisation tumorale globale a été obtenue pendant 20 jours avec la combinaison ECT+CpG de façon tout à fait significative avec le groupe témoin (z=2,47). Par contre les traitements simples n'ont pas permis de stabilisation mais ont été associés à une prolifération tumorale ralentie par rapport au groupe contrôle (Fig 1 B).

### - Analyse par souris :

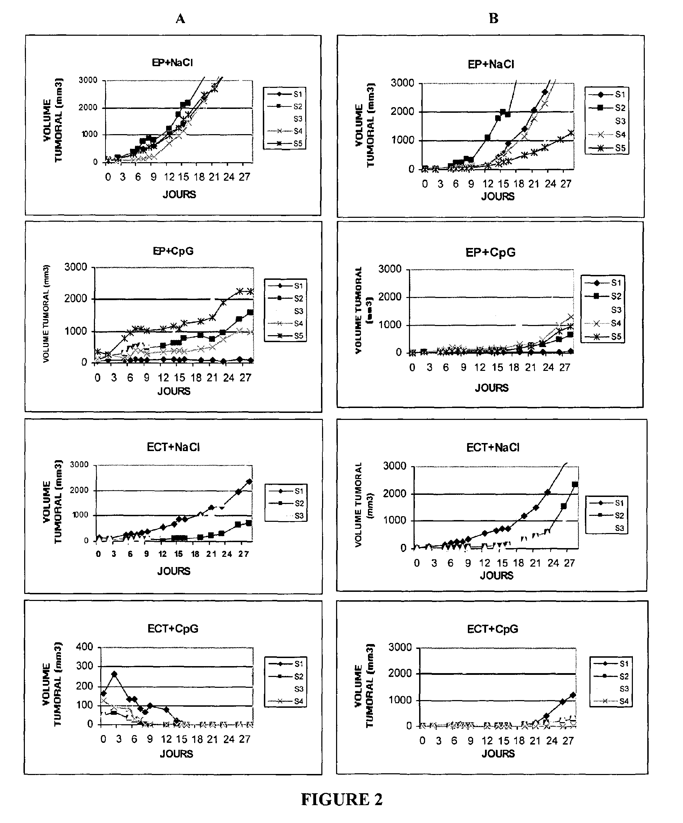

La représentation de la croissance des tumeurs, animal par animal, permet de suivre l'évolution des tumeurs gauche et droite chez une même souris (Fig. 2). L'évolution de la tumeur controlatérale semble être corrélée à l'évolution de la tumeur traitée sans que ce résultat soit généralisable à l'ensemble des souris. Par exemple, dans le groupe EP+CpG, la souris S1 ayant été stabilisée à gauche a aussi été stabilisée à droite alors que la souris S3 qui a évolué rapidement à gauche a progressé fortement à droite.

### • Modèle de mélanome B 160VA

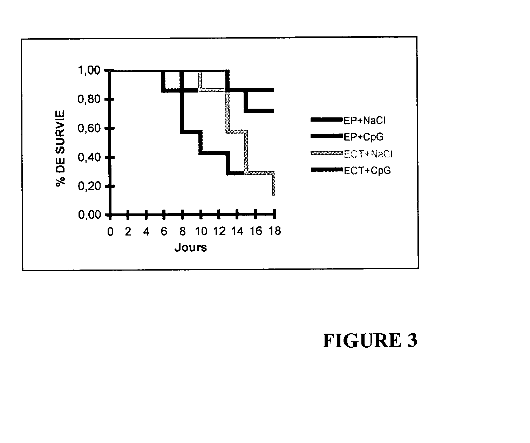

Le modèle de fibrosarcome a été reproduit en implantant également 10⁶ cellules B16OVA sur les flancs gauche et droit des souris et en respectant l'intervalle de 3 jours entre les deux injections. Les tumeurs ont été traitées comme décrit précédemment 9 jours après l'implantation des cellules. Une seule expérience actuellement en cours est décrite à partir du jour du début du traitement J0 (Fig. 3). Les taux de survie au 18^{ème} jour de traitement sont respectivement de 28,6 %, 71,4 %, 14,3 % et 85,7 % pour les groupes EP+NaCl, EP+CpG, ECT+NaCl et ECT+CpG.

### Analyse de l'effet de l'électroporation des CpG, dans le cadre d'une ECT :

Le système immunitaire inné détecte les motifs CpG non méthylés par les TLR9 chez la souris (26). Les TLR9 sont exprimés intracellulairement précisément au niveau du réticulum endoplasmique (30). L'élévation de la concentration intracellulaire des ODN-CpG permet d'augmenter leur activité stimulatrice (31). L'hypothèse d'une meilleure efficacité des ODN-CpG lorsqu'ils sont électroporés in *vivo* dans notre modèle de double tumeur LPB a été testée.

Deux groupes supplémentaires dans lesquels les CpG étaient électroporés dans la tumeur immédiatement après leur injection ont été menés. Les CpG étaient injectés en intratumoral puis électroporés à J1, J8, J15 et J22 comme précédemment.

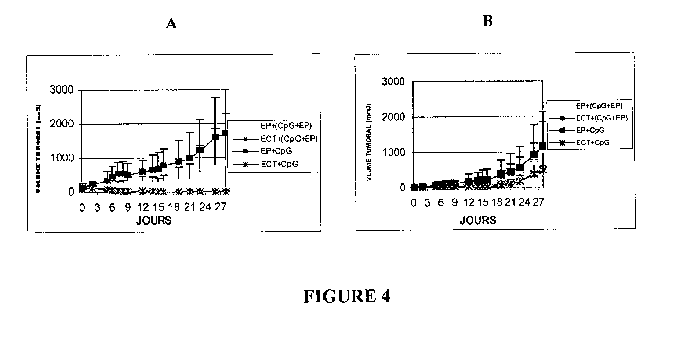

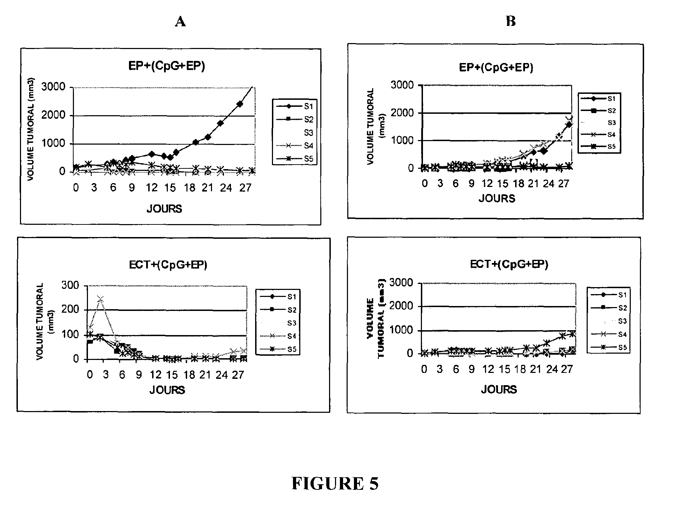

Le groupe des CpG électroporés combiné à l'ECT [ECT +(CpG+EP)] a montré une guérison des tumeurs traitées dans 100% des cas (Fig. 4A, 5A). Il n'y avait donc pas de différence d'effet antitumoral avec le groupe ECT+CpG qui, lui aussi, avait entraîné les mêmes résultats. Au contraire lorsqu'il n'y avait pas d'ECT, l'effet direct des CpG électroporés a montré une meilleure efficacité (sans différence significative) par rapport aux CpG non électroporés: 4 tumeurs sur 5 ont été stabilisées contre seulement 1 sur 5 (Fig. 4A, 5A).

Dans le groupe ECT+(CpG+EP) toutes les tumeurs controlatérales non traitées ont été stabilisées pendant 20 jours après l'ECT comme pour le groupe ECT+CpG (Fig. 4B, 5B). La croissance des tumeurs droites des groupes EP+(CpG+EP) et EP+CpG étaient sensiblement identiques.

Au niveau statistique le groupe ECT+(CpG+EP) était significativement différent à gauche avec les groupes EP+NaCl et EP+CpG. A droite ce groupe présentait une différence significative avec les groupes EP+NaCl et ECT+NaCl.

### Etude de l'induction de lymphocytes T cytotoxiques avec la combinaison ECT et CpG :

Pour tester la capacité de l'association thérapeutique à induire des lymphocytes T cytotoxiques spécifiques du peptide SIINFEKL, un modèle tumoral a été créé en injectant 500000 cellules B16OVA dans la cuisse des animaux. Ce modèle permettait de traiter facilement les animaux par ECT à J0 et CpG intratumoral à J1. Cinq groupes de 3 souris ont été créés : un groupe dont les souris sans tumeur ont été immunisées avec le peptide SIINFEKL mixé dans du CpG le jour de l'immunothérapie, trois groupes traités par ECT+CpG ou ECT+NaCl ou CpG seuls et un groupe non traité. Sept jours après l'injection des CpG les animaux ont été sacrifiés afin de prélever le ganglion inguinal homolatéral et controlatéral de chaque souris. La présence éventuelle de lymphocytes T cytotoxiques spécifiques dans le ganglion homolatéral a été recherchée *ex vivo* à l'aide d'un marquage par des tétramères solubles reconnaissant H-2^{b}/SIINFEKL. Les ganglions inguinaux controlatéraux nous servaient de contrôle interne négatif. Une seule expérience de ce type a été menée jusqu'à aujourd'hui mais une autre est en cours.

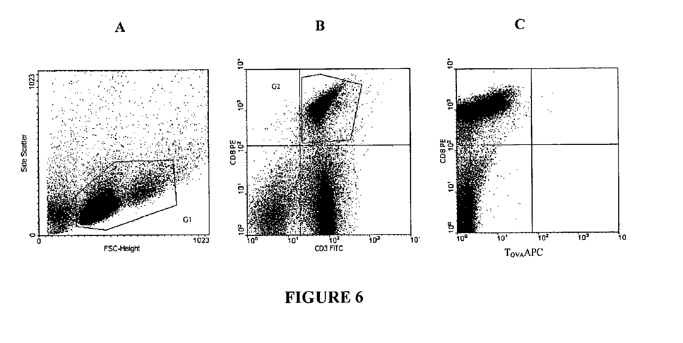

Une analyse en cytométrie de flux représentative de l'ensemble de tous les groupes est montrée dans la figure 6. Dans la population de cellules viables (fenêtre G1) nous avons sélectionné les lymphocytes T CD3/CD8 (fenêtre G2) dans lesquels le pourcentage de lymphocytes reconnus par le tétramère a été déterminé. Ce type d'analyse a été effectué pour tous les groupes de l'expérience, cependant aucun marquage positif n'a été détecté y compris dans le groupe contrôle immunisé par le peptide (Fig. 6C). On n'a pas retrouvé de sécrétion d'interféron γ dans le surnageant des cultures stimulées *in vitro* par le peptide.

Il ressort de l'ensemble de ces résultats que la combinaison ECT associée aux CpG a entraîné à la fois des effets locaux et à distance.

D'une part, on a constaté des régressions locales complètes dans presque 100% des cas (8 cas sur 9 dans l'ensemble des expériences) dans le modèle de fibrosarcome murin. Cette efficacité locale est supérieure à l'effet de chacun des deux traitements effectués séparément. La réduction tumorale importante induite par l'ECT semble donc être complétée par un effet antitumoral indirect dû à l'activation du système immunitaire par les CpG (action synergique), alors que l'électroporation seule et la bléomycine seule à des doses sub-thérapeutiques ne possèdent pas d'activité antitumorale propre (3).

D'autre part, concernant l'effet à distance de la combinaison ECT+CpG dans le modèle de fibrosarcome, 5 stabilisations de 20 jours et 3 régressions complètes sur 9 animaux dans l'ensemble des expériences ont été obtenues. Cette association met donc très probablement en jeu des mécanismes immunitaires responsables d'un effet systémique dans ce modèle. La mort cellulaire induite par l'ECT seule, même si elle semble s'associer à quelques effets immunologiques, n'est pas suffisamment immunogène pour entraîner un effet antitumoral à distance. Plusieurs hypothèses ont été faites pour expliquer pourquoi la mort cellulaire induite par l'ECT devenait immunogène lorsqu'elle était associée à l'administration de CpG, comme en témoignent les résultats.

L'observation détaillée des résultats animal par animal (Fig. 2) suggère une relation étroite entre l'effet antitumoral direct et l'effet à distance avec la nécessité, semble-t-il, pour obtenir un effet controlatéral d'avoir obtenu une activité locale et une fonte tumorale substantielle localement. Ainsi l'effet systémique du traitement paraît lié à sa capacité à induire une bonne réponse immunitaire locale. Comme suggéré précédemment concernant l'effet antitumoral local, la réponse immunitaire lymphocytaire T mise en place à gauche (tumeur traitée) pourrait être responsable de l'effet observé à distance.

La combinaison selon l'invention, qui cible la réponse immunitaire plus globalement et en amont en mettant en jeu à la fois l'immunité innée via la stimulation des NK et des macrophages et la charnière innée acquise en activant les CD permet d'aboutir à une réponse antitumorale efficace.

Le modèle de mélanome B 160VA a permis de tester l'efficacité de la combinaison selon l'invention sur un autre type de tumeur que le fibrosarcome permettant l'analyse de la réponse immunitaire grâce à l'expression du peptide SIINFEKL par la tumeur. Toutefois cette lignée tumorale s'est révélée très agressive et a entraîné des décès rapidement ne permettant pas d'évaluer l'évolution des tumeurs, dans le modèle de double tumeur, et donc de conclure quant aux effets locaux et distants du traitement. Néanmoins cette première expérience montrant un taux de survie supérieur aux autres groupes pour l'association ECT+CpG au 18^{ème} jour de traitement, encourage à mettre au point un modèle plus adapté pour analyser un effet thérapeutique local et systémique.

Par ailleurs, lorsque les CpG ont été électroporés (EP) afin de voir si l'accès des CpG à des compartiments intracellulaires différents avait un effet thérapeutique additionnel, des résultats comparables aux autres groupes sont apparus que ce soit au niveau local ou controlatéral. Dans l'ensemble des expériences sur le fibrosarcome LPB toutes les souris (n=9) du groupe ECT+(CpG+EP) ont régressé localement et ont répondu à distance (7 stabilisations et 2 régressions complètes). L'association ECT+CpG ayant montré aussi de très bons résultats localement (8 guérisons sur 9) et à distance (5 stabilisations et 3 guérisons, n=9), l'électroporation des CpG n'apporte pas plus d'efficacité. Le seul bénéfice notable a été observé dans le groupe où les CpG seuls ont été électroporés (EP+(CpG+EP)) dans lequel 4 stabilisations des tumeurs traitées sur 5 ont été observées, contre 1 sur 5 dans le groupe traité par CpG seul (EP+CpG). *In vitro,* l'électroporation des CpG entraîne 20 à 30% de mortalité sur les cellules tumorales sans effet dose. Cela est peut-être dû à un phénomène de saturation de la cytotoxicité cellulaire ou de pénétration limitée dans les cellules. *In vivo* le retentissement de la cytotoxicité directe des CpG électroporés est difficilement appréciable. De plus dans ces expériences la tumeur était électroporée 4 fois de suite sans que l'on sache exactement quelles conséquences cela entraîne sur la croissance.

### BIBLIOGRAPHIE

1. Orlowski, S., and L. M. Mir. 1993. Cell electropermeabilization: a new tool for biochemical and pharmacological studies. *Biochim Biophys Acta 1154: 51.*
2. Mir, L. M., H. Banoun, and C. Paoletti. 1988. Introduction of definite amounts of nonpermeant molecules into living cells after electropermeabilization: direct access to the cytosol. *Exp Cell Res 175:15-25.*
3. Mir, L. M., S. Orlowski, J. Belehradek, Jr., and C. Paoletti. 1991. Electrochemotherapy potentiation of antitumour effect of bleomycin by local electric pulses. *Eur J Cancer 27: 68.*
4. Mir, L. M., O. Tounekti, and S. Orlowski. 1996. Bleomycin: revival of an old drug. *Gen Pharmacol 27: 745.*
5. Poddevin, B., S. Orlowski, J. Belehradek, Jr., and L. M. Mir. 1991. Very high cytotoxicity of bleomycin introduced into the cytosol of cells in culture. *Biochem Pharmacol 42 Suppl:S67.*
6. Tounekti, O., G. Pron, J. Belehradek, Jr., and L. M. Mir. 1993. Bleomycin, an apoptosis-mimetic drug that induces two types of cell death depending on the number of molecules internalized. *Cancer Res 53:5462.*
7. Mekid, H., O. Tounekti, A. Spatz, M. Cemazar, F. Z. El Kebir, and L. M. Mir. 2003. In vivo evolution of tumour cells after the generation of double-strand DNA breaks. *Br J Cancer 88:1763.*
8. Okino, M., and H. Mohri. 1987. Effects of a high-voltage electrical impulse and an anticancer drug on in vivo growing tumors. *Jpn J Cancer Res 78:1319.*
9. Ramirez, L. H., S. Orlowski, D. An, G. Bindoula, R. Dzodic, P. Ardouin, C. Bognel, J. Belehradek, Jr., J. N. Munck, and L. M. Mir. 1998. Electrochemotherapy on liver tumours in rabbits. *Br J Cancer 77:2104.*
10. Mir, L. M., and S. Orlowski. 1999. Mechanisms of electrochemotherapy. Adv *Drug Deliv Rev 35:107.*
11. Sersa, G., D. Miklavcic, M. Cemazar, J. Belehradek, T. Jarm and L.M. Mir. 1997. Electrochemotherapy with CDDP on LPB sarcoma: comparison of the anti-tumor effectiveness in immunocompetent and immunideficient mice. *Bioelectrochemistry and Bioenergetics 43:279.*
12. Mir, L. M., S. Orlowski, B. Poddevin, and J. Belehradek, Jr. 1992. Electrochemotherapy tumor treatment is improved by interleukin-2 stimulation of the host's defenses. *Eur Cytokine Netw 3:331.*
13. Belehradek, M., C. Domenge, B. Luboinski, S. Orlowski, J. Belehradek, Jr., and L. M. Mir. 1993. Electrochemotherapy, a new antitumor treatment. First clinical phase I-II trial. *Cancer 72:3694.*
14. Heller, R., R. Gilbert, and M. J. Jaroszeski. 1999. Clinical applications of electrochemotherapy. *Adv Drug Deliv Rev 35:119.*
15. Gothelf, A., L. M. Mir, and J. Gehl. 2003. Electrochemotherapy: results of cancer treatment using enhanced delivery of bleomycin by electroporation. *Cancer Treat Rev 29: 371.*
16. Sersa, G., M. Cemazar, V. Menart, V. Gaberc-Porekar, and D. Miklavcic. 1997. Anti-tumor effectiveness of electrochemotherapy with bleomycin is increased by TNF-alpha on SA-1 tumors in mice. *Cancer Leit 116:85.*
17. Mir, L. M., C. Roth, S. Orlowski, F. Quintin-Colonna, D. Fradelizi, J. Belehradek, Jr., and P. Kourilsky. 1995. Systemic antitumor effects of electrochemotherapy combined with histoincompatible cells secreting interleukin-2. J *Immunother Emphasis Tumor Immunol 17:30.*
18. Mir, L. M., C. Roth, S. Orlowski, J. Belehradek, Jr., D. Fradelizi, C. Paoletti, and P. Kourilsky. 1992. [Potentiation of the antitumoral effect of electrochemotherapy by immunotherapy with allogeneic cells producing interleukin 2]. *C R Acad Sci III 314:539.*
19. Roth, C., L. M. Mir, M. Cressent, F. Quintin-Colonna, V. Ley, D. Fradelizi, and P. Kourilsky. 1992. Inhibition of tumor growth by histoincompatible cells expressing interleukin-2. *Int Immunol 4:1429.*
20. Orlowski, S., D. An, J. Belehradek, Jr., and L. M. Mir. 1998. Antimetastatic effects of electrochemotherapy and of histoincompatible interleukin-2-secreting cells in the murine Lewis lung tumor. *Anticancer Drugs 9:551.*
21. Andersen, M. H., J. Gehl, S. Reker, L. O. Pedersen, J. C. Becker, P. Geertsen, and P. thor Straten. 2003. Dynamic changes of specific T cell responses to melanoma correlate with IL-2 amdministration. *Semin Cancer Biol 13:449.*
22. Gilboa, E. 1999. The makings of a tumor rejection antigen. *Immunity 11:263.*
23. Dhodapkar, M. V., J. W. Young, P. B. Chapman, W. I. Cox, J. F. Fonteneau, S. Amigorena, A. N. Houghton, R. M. Steinman, and N. Bhardwaj. 2000. Paucity of functional T-cell memory to melanoma antigens in healthy donors and melanoma patients. *Clin Cancer Res 6*: *4831.*
24. Tokunaga, T., H. Yamamoto, S. Shimada, H. Abe, T. Fukuda, Y. Fujisawa, Y. Furutani, O. Yano, T. Kataoka, T. Sudo, and et al. 1984. Antitumor activity of deoxyribonucleic acid fraction from Mycobacterium bovis BCG. I. Isolation, physicochemical characterization, and antitumor activity. *J Natl Cancer Inst 72:955.*
25. Krieg, A. M., A. K. Yi, S. Matson, T. J. Waldschmidt, G. A. Bishop, R. Teasdale, G. A. Koretzky, and D. M. Klinman. 1995. CpG motifs in bacterial DNA trigger direct B-cell activation. *Nature 374:546.*
26. Hemmi, H., O. Takeuchi, T. Kawai, T. Kaisho, S. Sato, H. Sanjo, M. Matsumoto, K. Hoshino, H. Wagner, K. Takeda, and S. Akira. 2000. A Toll-like receptor recognizes bacterial DNA. *Nature 408: 740.*
27. Miconnet, I., S. Koenig, D. Speiser, A. Krieg, P. Guillaume, J. C. Cerottini, and P. Romero. 2002. CpG are efficient adjuvants for specific CTL induction against tumor antigen-derived peptide. *J Immunol 168.-1212.*
28. Carpentier, A. F., L. Chen, F. Maltonti, and J. Y. Delattre. 1999. Oligodeoxynucleotides containing CpG motifs can induce rejection of a neuroblastoma in mice. *Cancer Res 59:5429.*
29. Heckelsmiller, K., K. Rall, S. Beck, A. Schlamp, J. Seiderer, B. Jahrsdorfer, A. Krug, S. Rothenfusser, S. Endres, and G. Hartmann. 2002. Peritumoral CpG DNA elicits a coordinated response of CD8 T cells and innate effectors to cure established tumors in a murine colon carcinoma model. *J Immunol 169: 3892.*
30. Latz, E., A. Schoenemeyer, A. Visintin, K. A. Fitzgerald, B. G. Monks, C. F. Knetter, E. Lien, N. J. Nilsen, T. Espevik, and D. T. Golenbock. 2004. TLR9 signals after translocating from the ER to CpG DNA in the lysosome. *Nat Immunol 5:190.*
31. Yamamoto, T., S. Yamamoto, T. Kataoka, and T. Tokunaga. 1994. Lipofection of synthetic oligodeoxyribonucleotide having a palindromic sequence of AACGTT to murine splenocytes enhances interferon production and natural killer activity. *Microbiol Immunol 38:831.*
32. Auf, G., A. F. Carpentier, L. Chen, C. Le Clanche, and J. Y. Delattre. 2001. Implication of macrophages in tumor rejection induced by CpG-oligodeoxynucleotides without antigen. *Clin Cancer Res 7:3540.*
33. Ballas, Z. K., W. L. Rasmussen, and A. M. Krieg. 1996. Induction of NK activity in murine and human cells by CpG motifs in oligodeoxynucleotides and bacterial DNA. *J Immunol 157:1840.*
34. J. Belehradek Jr, S. Orlowski, B. Poddevin, C. Paoletti and L.M. Mir. Electrochemotherapy of spontaneous mammary tumours in mice. European Journal of Cancer, 27, 73-76 (1991).
35. C.Domenge, S.Orlowski, B.Luboinski, T. De Baere, G. Schwaab, J.Belehradek Jr and L.M.Mir. Antitumor electrochemotherapy : new advances in the clinical protocols. Cancer 77, 956-963, 1996.
36. J.Gehl, T.Skovsgaard and L.M. Mir. Enhancement of cytotoxicity by electropermeabilization: an improved method for screening drugs. Anti-Cancer Drugs 9, 319-325, 1998.
37. T. Kotnik, G. Pucihar, M. Rebersek, D. Miklav 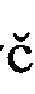 i 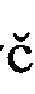 and L.M. Mir. The role of pulse shape in cell membrane electropermeabilization *in vitro.* Biochimica Biophysica Acta - Biomembranes, 1614, 193-200, 2003.
38. L.M.Mir, M.Belehradek, C.Domenge, S.Orlowski, B.Poddevin, J.Belehradek Jr, G.Schwaab, B.Luboinski and C.Paoletti. L'électrochimiothérapie, un nouveau traitement antitumoral : premier essai clinique - Electrochemotherapy, a novel antitumor treatment : first clinical trial. Compte rendus de l'Académie des Sciences, sér III, 313, 613-618 (1991b).
39. L.M. Mir, F.L. Glass, G.Sersa, J.Teissié, C.Domenge, D. Miklavcic, M.J. Jaroszeski, S. Orlowski, D.S. Reintgen, Z. Rudolf, M. Belehradek, R. Gilbert, M.P. Rols, J. Belehradek Jr, J.M. Bachaud, R. DeConti, B. Stabuc, P. Coninx, M. Cemazar, R.Heller. Effective treatment of cutaneous and subcutaneous malignant tumors by electrochemotherapy. British Journal of Cancer 77, 2336-2342, 1998.
40. Orlowski S, Belehradek J, Jr., Paoletti C, Mir LM. Transient electropermeabilisation of cells in culture. Increase of the cytotoxicity of anticancer drugs. *Biochem Pharmacol 37:* 4727-4733, 1988.
41. Sersa G, Cemazar M, Rudolf Z. Electrochemotherapy: advantages and drawbacks in treatment of cancer patients. *Cancer Therapy 1:* 133-142, 2003.
42. Teissié J., Rols M. P. An experimental evaluation of the critical potential difference inducing cell membrane électropermeabilization. *Biophys J,* 65:409-13, 1993.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Produits contenant au moins un principe actif anticancéreux peu diffusible et un principe actif immunostimulant sélectionné dans le groupe constitué par des ligands de TLR en tant que préparation combinée pour une utilisation simultanée, séparée ou étalée dans le temps, pour le traitement des tumeurs bénignes ou malignes et plus particulièrement des métastases, chez un sujet atteint d'une tumeur et soumis à une électroperméabilisation au niveau de ladite tumeur.

2. Produits selon la revendication 1, **caractérisés en ce que** les ligands de TLR sont des oligodésoxynucléotides (ODN) immunostimulants.

3. Produits selon la revendication 1 ou la revendication 2, **caractérisés en ce que** les ligands de TLR sont des ligands de TLR9.

4. Produits selon la revendication 3, **caractérisés en ce que** les ligands de TLR9 sont des CpG-ODN immunostimulants.

5. Produits selon la revendication 4, **caractérisés en ce que** le CpG-ODN immunostimulant est sélectionné dans le groupe constitué par un oligodésoxynucléotide stabilisé qui comprend au moins un motif quadramérique de formule X₁-CG-X₂, dans laquelle X₁ et X₂ sont identiques ou différents et représentent T ou A.

6. Produits selon la revendication 5, **caractérisés en ce que** ledit CpG-ODN est de préférence sélectionné dans le groupe constitué par un oligodésoxynucléotide qui comprend au moins une séquence quadramérique sélectionnée dans le groupe constitué par : TCGA, ACGT, ACGA et TCGT.

7. Produits selon la revendication 5 ou la revendication 6, **caractérisés en ce que** ledit CpG-ODN est de préférence sélectionné dans le groupe constitué par un oligodésoxynucléotide qui comprend au moins une séquence hexamérique sélectionnée dans le groupe constitué par : AACGTT, GACGTC, GACGTT, GTCGTT, TTCGAA, TACGTA et ATCGAT.

8. Produits selon la revendication 7, **caractérisés en ce que** ledit CpG-ODN est de préférence sélectionné dans le groupe constitué par un oligonucléotide qui comprend au moins la séquence octamérique suivante : AACGTT-X₃X₄, dans laquelle X₃X₄ est AT, AA, CT ou TT et de préférence la séquence octamérique AACGTTAT.

9. Produits selon l'une quelconque des revendications 5 à 9, **caractérisés en ce que** ledit CpG-ODN est le CpG-ODN de séquence SEQ ID NO: 1.

10. Produits selon l'une quelconque des revendications 1 à 9, **caractérisés en ce que** le principe actif anticancéreux est sélectionné dans le groupe constitué par les substances cytotoxiques sélectionnées dans le groupe constitué par les antimétabolites et les substances génotoxiques.

11. Produits selon l'une quelconque des revendications 1 à 10, **caractérisés en ce que** le principe actif anticancéreux est de préférence une substance génotoxique et de manière encore plus préférée de la bléomycine.

12. Produits selon l'une quelconque des revendications 1 à 11, **caractérisés en ce que** le principe actif anticancéreux est administré par voie intratumorale, locale ou systémique.

13. Produits selon l'une quelconque des revendications 1 à 12, **caractérisés en ce que** le principe actif immunostimulant est administré par voie intratumorale, locale ou systémique.

14. Produits selon l'une quelconque des revendications 1 à 13, **caractérisés en ce que** les cellules de la tumeur sont électroperméabilisées par exposition desdites cellules tumorales à des impulsions électriques de force ou d'intensité et de durée suffisantes pour permettre l'électroporation desdites cellules cancéreuses.

15. Produits selon la revendication 14, **caractérisés en ce que** l'intensité du champ électrique appliqué varie entre 20 et 2 000 V/cm.

16. Produits selon la revendication 15, **caractérisés en ce que** l'intensité du champ électrique appliqué varie entre 500 et 1 500 V/cm.

17. Produits selon la revendication 14, **caractérisés en ce que** la fréquence des impulsions varie de 0,01 à 10 000 Hz.

18. Produits selon la revendication 17, **caractérisés en ce que** la fréquence des impulsions varie de 1 à 5 000 Hz.

19. Produits selon la revendication 14, **caractérisés en ce que** la durée des impulsions varie de 10 µs à 10 s.

20. Produits selon la revendication 19, **caractérisés en ce que** la durée des impulsions varie de 50 µs à 500 ms.

21. Produits contenant au moins un principe actif anticancéreux peu diffusible, un principe actif immunostimulant sélectionné dans le groupe constitué par des ligands de TLR et un antigène convenable en tant que préparation combinée pour une utilisation simultanée, séparée ou étalée dans le temps, pour le traitement des tumeurs bénignes ou malignes et plus particulièrement des métastases, chez un sujet atteint d'une tumeur et soumis à une électroperméabilisation au niveau d'un tissu sain sélectionné de manière appropriée.

22. Produits selon la revendication 21, **caractérisés en ce que** ledit antigène est sélectionné dans le groupe constitué par les antigènes purifiés spécifiques de tumeurs, des extraits tumoraux, des cellules tumorales irradiées ou modifiées.


LISTE DE SEQUENCES

<110>   INSTITUT GUSTAVE ROUSSY
        CENTRE NATIONAL DE LA RECHERCHE SCIENTIFIQUE
        UNIVERSITE RENE DESCARTES - PARIS 5
        OLIGOVAX

<120> Produits contenant au moins un principe actif anticancéreux peu
diffusible et un principe actif immunostimulant


<130> 1534EP6

<140>
<141>

<160> 1

<170> PatentIn Ver. 2.1

<210> 1
<211> 26
<212> ADN
<213> Séquence artificielle

<220>
<223> Description de la séquence artificielle:
      oligodésoxynucléotide

<400> 1
taaacgttat aacgttatga cgtcat                                       26